# EUROPEAN PATENT APPLICATION

(11) **EP 3 184 055 A1**
(43) Date of publication of application: **28.06.2017**
(21) Application number: 15833353.4
(22) Date of filing: 01.05.2015
(51) Int. Cl.: A61B 10/06, A61B 1/00

(54) **MEDICAL TREATMENT INSTRUMENT**

(30) Priority: 19.08.2014 JP 2014166318
(71) Applicant: OLYMPUS CORPORATION, Hachioji-shi Tokyo 192-8507 (JP)
(72) Inventor: TAKAHASHI, Ichiro, Tokyo 1928507 (JP)
(74) Representative: Schicker, Silvia
(86) International application number: PCT/JP2015/063098
(87) International publication number: WO 2016/027523

(57) **Abstract**

A medical treatment instrument includes a sheath, a needle advanceably and retractably inserted into the sheath, and a stylet having a first outer diameter and advanceably and retractably inserted into the needle. A distal end portion of the needle includes a housing portion, a small-diameter portion provided at a proximal end of the housing portion and having an inner diameter smaller than that of the housing portion, and a slit extending at least from a distal end of the housing portion to a proximal end of the small-diameter portion. A distal end portion of the stylet includes a large-diameter portion having a second outer diameter larger than the first outer diameter. The small-diameter portion and the housing portion are elastically deformed to expand in a radial direction of the needle while a width of the slit expands by the large-diameter portion being engaged with the small-diameter portion.

## Description

### Technical Field

The present invention relates to a medical treatment instrument, and more particularly, to a medical treatment instrument which is inserted into a body cavity through a treatment instrument channel of an endoscope to collect tissue or a cell at a predetermined portion of the body cavity.

Priority is claimed on Japanese Patent Application No. 2014-166318, filed August 19, 2014, the content of which is incorporated herein by reference.

### Background Art

Conventionally, as a treatment instrument which performs treatment of tissue or a cell at a predetermined portion of a body cavity, for example, a treatment instrument described in Patent Literature 1 is known. In addition, in Patent Literature 2, a treatment instrument which performs treatment of a submucosal tumor is disclosed. The treatment instrument described in Patent Literature 2 includes a sheath, an operation motion transmission member which is disposed to be capable of advancing and retracting in its axial direction along the sheath to advance and retract by an operation from a hand side, and a pair of distal end treatment members disposed to be openable and closable in the shape of a beak at a distal end of the sheath to be opened and closed by the advancing and retracting movement of the operation motion transmission member. In the treatment instrument, distal end portions of the pair of distal end treatment members are formed in acute shapes to be sharp at the front. By the treatment instrument, puncture of a submucosal tumor and collection of a biopsy tissue can be performed in one operation.

In addition, as a method of collecting tissue of a submucosal tumor, aspiration biopsy which is performed by Fine-Needle Aspiration (FNA), for example, is known. During the aspiration biopsy, tissue is punctured with a biopsy needle, and then the tissue is aspirated by filling a negative pressure in the biopsy needle with a syringe, and so on.

### Citation List

### Patent Literature

Patent Literature 1: Japanese Unexamined Patent Application, First Publication No. 2008-5965
Patent Literature 2: Japanese Unexamined Patent Application, First Publication No. 2000-201939

### Summary of Invention

### Technical Problem

To properly puncture tissue in a body with a needle, in general, an outer diameter of the needle is set to be maximally about 1 mm. This is also the same for the treatment instrument described in Patent Literature 2, and outer diameters of the pair of distal end treatment members are set in consideration of the limitation above. In addition, a link mechanism used in opening and closing the pair of distal end treatment members is also constituted in a small size due to the limitation above. When the treatment instrument described in Patent Literature 2 is used to collect tissue, the pair of distal end treatment members has to be closed while the tissue is split by the pair of distal end treatment members. However, when collecting tissue of a submucosal tumor which is hard tissue, the link mechanism does not have sufficient power to split the tissue due to its small size, and thus there may be a case in which the power cannot overcome the hardness of the tissue so that the pair of distal end treatment members cannot be sufficiently closed. In this case, it may not be possible to collect an amount of tissue sufficient for diagnosing the tissue.

In addition, in a case of aspiration biopsy, an outer diameter of a biopsy needle is narrow as described above, and tissue can be aspirated only within a region defined by an inner diameter of the biopsy needle. In addition, since the biopsy needle does not have a structure in which tissue is actively introduced into its inner space, even when tissue of a submucosal tumor which is hard tissue is aspirated, there are cases in which the tissue is not collected in the biopsy needle at all or only a very small amount of the tissue is collected. Thus, using the aspiration biopsy, there are cases in which it is difficult to collect an amount of tissue sufficient for diagnosing the tissue.

An object of the present invention is to provide a medical treatment instrument capable of properly puncturing tissue and collecting a sufficient amount of the tissue from within the tissue.

### Solution to Problem

According to a first aspect of the present invention, a medical treatment instrument includes: a sheath; a needle formed in a tubular shape and inserted into the sheath such that the needle is capable of advancing and retracting; and a stylet having a first outer diameter and inserted into the needle such that the stylet is capable of advancing and retracting. A distal end portion of the needle is elastically deformable. The distal end portion of the needle includes: a housing portion having a space formed therein; a small-diameter portion provided at a proximal end of the housing portion and having an inner diameter smaller than an inner diameter of the housing portion; and a slit extending at least from a distal end of the housing portion to a proximal end of the small-diameter portion and communicating with the space. A distal end portion of the stylet includes a large-diameter portion having a second outer diameter which is larger than the first outer diameter. By the large-diameter portion being engaged with the small-diameter portion, the small-diameter portion and the housing portion are elastically deformed to expand in a radial direction of the needle while a width of the slit expands.

According to a second aspect of the present invention, in the medical treatment instrument according to the first aspect, the first outer diameter may be smaller than the inner diameter of the small-diameter portion. The second outer diameter may be larger than the inner diameter of the small-diameter portion and is smaller than the inner diameter of the housing portion.

According to a third aspect of the present invention, in the medical treatment instrument according to the first aspect, the slit may include a plurality of slits.

According to a fourth aspect of the present invention, in the medical treatment instrument according to the first aspect, the large-diameter portion may be positioned closer to a distal end side than the small-diameter portion in an initial state, and the large-diameter portion may expand the small-diameter portion by the stylet being pulled toward a proximal end side with respect to the needle.

### Advantageous Effects of Invention

According to a medical treatment instrument according to each of the above aspects, since the housing portion of the needle can expand in the radial direction of the needle by elastic deformation, even when an outer diameter of the needle is narrow to an extent that the needle can properly puncture tissue, a sufficient amount of the tissue can be collected by the expansion of the housing portion.

### Brief Description of Drawings

Fig. 1 is a view showing an endoscope used together with a medical treatment instrument according to a first embodiment of the present invention.
Fig. 2 is a lateral view of the medical treatment instrument.
Fig. 3 is a lateral cross-sectional view of the medical treatment instrument.
Fig. 4 is a view showing the medical treatment instrument viewed from an arrow A in Fig. 2.
Fig. 5 is a lateral cross-sectional view of a distal end portion of the medical treatment instrument.
Fig. 6 is a lateral cross-sectional view when the distal end portion of the medical treatment instrument expands.
Fig. 7 is a view showing an operation when the medical treatment instrument is being used.
Fig. 8 is a view showing an operation when the medical treatment instrument is being used.
Fig. 9 is a view showing an operation when the medical treatment instrument is being used.
Fig. 10 is a view showing an operation when the medical treatment instrument is being used.
Fig. 11 is a lateral cross-sectional view of a distal end portion of a medical treatment instrument according to a second embodiment of the present invention.
Fig. 12 is a view showing a state in which a distal end portion of a needle of the medical treatment instrument is closed.
Fig. 13 is a view showing a state in which the distal end portion of the needle of the medical treatment instrument is open.
Fig. 14 is a view showing the needle viewed from an arrow B in Fig. 12.
Fig. 15 is a view showing an operation when the medical treatment instrument is being used.
Fig. 16 is a view showing an operation when the medical treatment instrument is being used.
Fig. 17 is a view showing an operation when the medical treatment instrument is being used.

### Description of Embodiments

### (First embodiment)

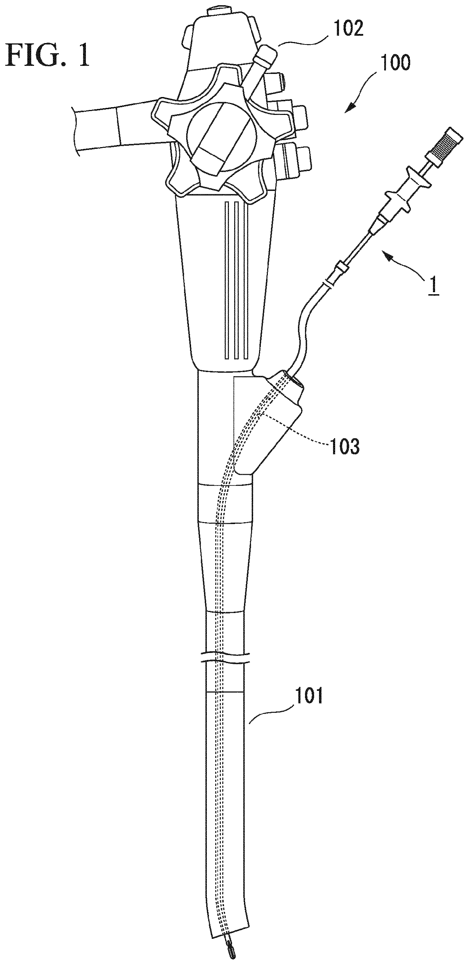

Hereinafter, a first embodiment of the present invention will be described with reference to Figs. 1 to 10. Fig. 1 is a view showing an endoscope 100 used together with a medical treatment instrument 1 according to the embodiment.

As shown in Fig. 1, the endoscope 100 includes an endoscope insertion part 101 inserted into a body, and an operation part 102 which operates the endoscope insertion part 101. The operation part 102 is provided at a proximal end of the endoscope insertion part 101. The operation part 102 bends a distal end portion of the endoscope insertion part 101 by an angular wire (not shown). At a distal end of the endoscope insertion part 101, a known imaging means which observes an inside of the body is provided. A channel 103 is formed inside the endoscope insertion part 101. The channel 103 extends from the distal end of the endoscope insertion part 101 to a portion near a side part of the operation part 102. An opening of a distal end of the channel 103 is formed at the distal end of the endoscope insertion part 101, and an opening of a proximal end of the channel 103 is formed at the portion near the side part of the operation part 102. The opening of the proximal end of the channel 103 is an insertion port of a treatment instrument which is inserted into the endoscope 100. The medical treatment instrument 1 according to the embodiment is inserted into the channel 103 through the insertion port.

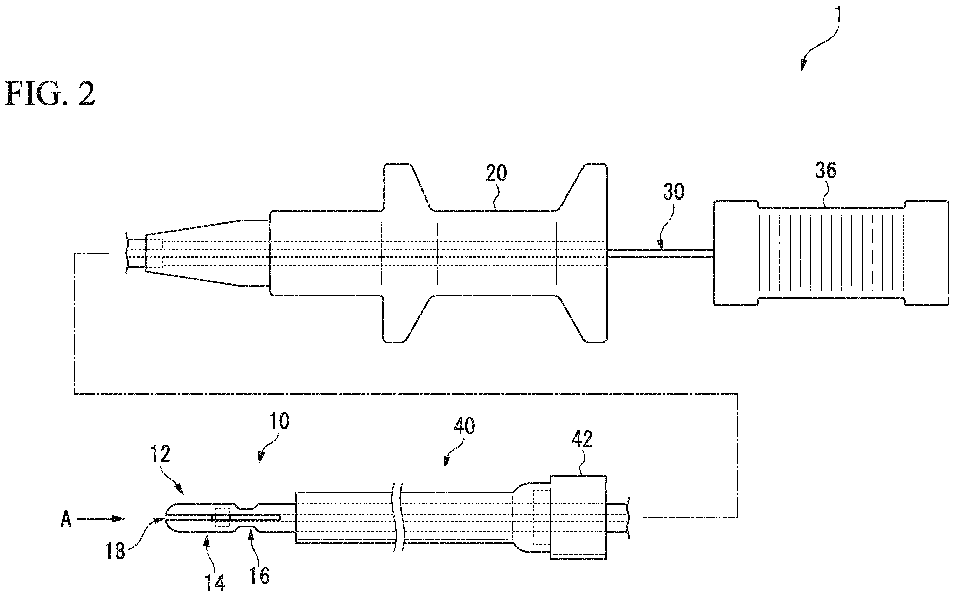

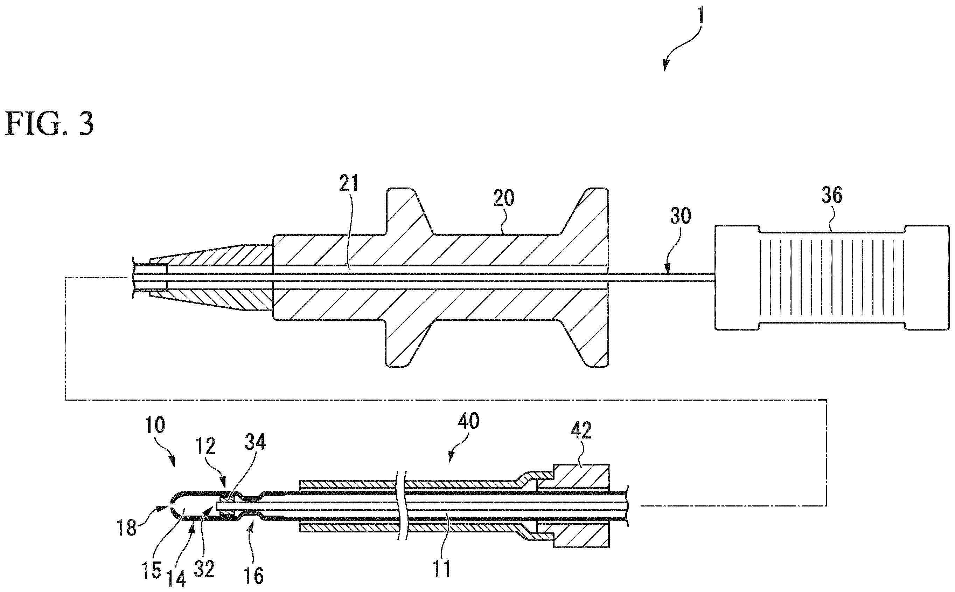

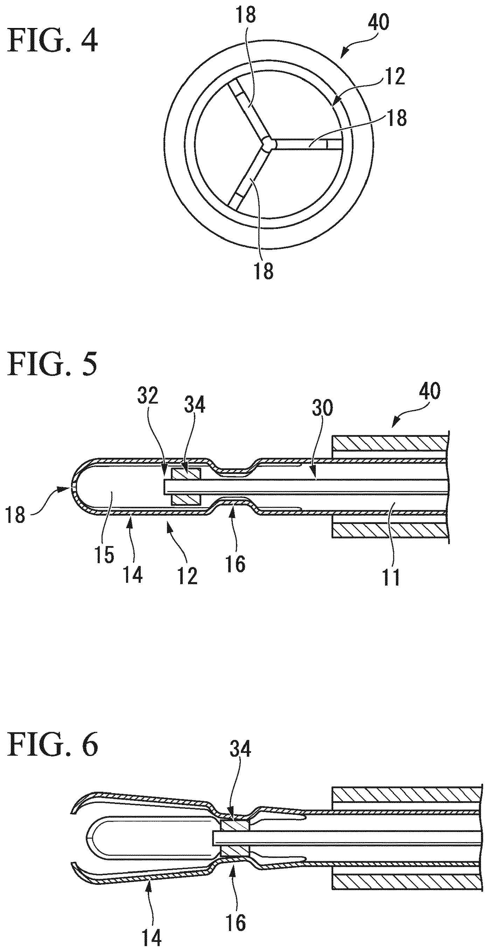

Fig. 2 is a lateral view of the medical treatment instrument 1 according to the embodiment. Fig. 3 is a lateral cross-sectional view of the medical treatment instrument 1. Fig. 4 is a view showing the medical treatment instrument 1 viewed from an arrow A in Fig. 2. Fig. 5 is a lateral cross-sectional view of a distal end portion of the medical treatment instrument 1. As shown in Fig. 2, the medical treatment instrument 1 includes a needle 10, a stylet 30, and a sheath 40. The needle 10 is formed in a tubular shape and is inserted into the sheath 40 to be capable of advancing and retracting. The stylet 30 has a predetermined outer diameter (first outer diameter) and is inserted into the needle 10 to be capable of advancing and retracting.

The needle 10 is constituted with a metal such as stainless steel or a nickel-titanium alloy and can be elastically deformed. The needle 10 is formed in the tubular shape and has a lumen 11. Like a general puncturing needle, the needle 10 may be, for example, a 19-gauge needle (outer diameter of 1.1 mm, inner diameter of 0.78 mm). In addition, a needle which is narrower than the needle having the size above may also be used as the needle 10. A distal end of the needle 10 is formed in the shape of a curved surface so as to easily puncture tissue with the needle 10. In addition, the distal end of the needle 10 may also be formed in an acute shape.

A distal end portion 12 of the needle 10 can be elastically deformed. As shown in Fig. 5, the distal end portion 12 of the needle 10 has a housing portion 14, a small-diameter portion 16, and a slit 18. A space 15 is formed in the housing portion 14. The small-diameter portion 16 is provided at a proximal end of the housing portion 14, and has a smaller inner diameter than an inner diameter of the housing portion 14. The slit 18 extends at least from a distal end of the housing portion 14 to a proximal end of the small-diameter portion 16, and communicates with the space 15.

The space 15 capable of accommodating tissue to be collected is formed in the housing portion 14. The inner diameter and the outer diameter of the housing portion 14 are substantially the same as the inner diameter and the outer diameter of the needle 10 which is closer to the proximal end side than the distal end portion 12, respectively. However, the inner diameter and the outer diameter of the housing portion 14 may respectively be set differently from the inner diameter and the outer diameter of the needle 10 which is closer to the proximal end side than the distal end portion 12. The length of the housing portion 14 in an axial direction of the needle 10 is properly set depending on an amount of tissue to be collected. The size of the space 15 is defined by the inner diameter of the housing portion 14 and the length of the housing portion 14 in the axial direction of the needle 10.

The small-diameter portion 16 is provided at the proximal end of the housing portion 14. The small-diameter portion 16 has a smaller inner diameter than other portions of the needle 10 including the housing portion 14. The small-diameter portion 16 may be formed by making a part of a proximal end side of the housing portion 14 in the needle 10 concave in a radial direction of the needle 10. In this case, the small-diameter portion 16 has a smaller outer diameter than other portions of the needle 10 including the housing portion 14. A proximal end of the small-diameter portion 16 continues to a straight tube portion of the needle 10. The straight tube portion of the needle 10 is formed in the shape of a tube with a fixed inner diameter and a fixed outer diameter.

The slit 18 is formed at the distal end portion 12, and extends from the distal end of the needle 10 (distal end of the housing portion 14) to the proximal end of the distal end portion 12. The slit 18 should extend at least from the distal end of the needle 10 to the proximal end of the small-diameter portion 16. In the embodiment, an example in which three slits 18 are formed is shown. However, the number of slits is not limited as long as there are one or more slits. When a plurality of slits 18 are formed, as in the embodiment shown in Fig. 4, the slits 18 are preferably formed at equal intervals in a circumferential direction of the needle 10. The slits 18 communicate with the lumen 11 and the space 15. In addition, the slits 18 intersect with or join each other at the distal end of the needle 10. Thus, the distal end portion 12 is divided into three portions by the slits 18. The widths of the slits 18 in the circumferential direction of the needle 10 are not particularly limited as long as the distal end portion 12 is divided by the slits 18. In order to prevent foreign substances other than target tissue from being mixed in the space 15, the widths of the slits 18 are preferably as narrow as possible. In addition, in the embodiment, although the slits 18 are formed in linear shapes along a longitudinal axis of the needle 10, the shapes of the slits 18 are not limited thereto. For example, the slits 18 may be formed in linear shapes inclined with respect to the longitudinal axis of the needle 10 or curved shapes.

As shown in Figs. 2 and 3, a needle operation part 20 is provided at the proximal end of the needle 10. Inside the needle operation part 20, an insertion passage 21 which communicates with the lumen 11 is formed. By advancing and retracting the needle operation part 20, the needle 10 can advance and retract with respect to the sheath 40.

The stylet 30 has the predetermined outer diameter (first outer diameter), and is inserted into the needle 10 to be capable of advancing and retracting. The stylet 30 is constituted with metal such as stainless steel, and is formed in the shape of a round rod. The stylet 30 is inserted into the lumen 11 and the insertion passage 21, and is capable of advancing and retracting with respect to the needle 10. The outer diameter (first outer diameter) of the stylet 30 is smaller than the inner diameter of the small-diameter portion 16.

A distal end portion 32 of the stylet 30 has a large-diameter portion 34. The large-diameter portion 34 is formed in a substantially cylindrical shape, and has an outer diameter (second outer diameter) larger than the outer diameter (first outer diameter) of other portions of the stylet 30. The outer diameter (second outer diameter) of the large-diameter portion 34 is larger than the inner diameter of the small-diameter portion 16, and is smaller than the inner diameter of the housing portion 14.

A stylet operation part 36 is provided at a proximal end of the stylet 30. By advancing and retracting the stylet operation part 36, the stylet 30 can advance and retract with respect to the needle 10.

The sheath 40 is constituted with a resin material, and is formed in a tubular shape. In addition, the sheath 40 may be formed by a coil sheath around which a wire of a metal material such as stainless steel is tightly wound with no gaps formed therebetween. The needle 10 is inserted into a lumen of the sheath 40 to be capable of advancing and retracting with respect to the sheath 40. When the medical treatment instrument 1 is inserted into the channel 103, the distal end of the needle 10 is stored in the sheath 40. In this way, damage of the needle 10 or the channel 103 due to direct contact between the needle 10 and an inner surface of the channel 103 is prevented.

A sheath operation part 42 is provided at a proximal end of the sheath 40. By advancing and retracting the sheath operation part 42, the sheath 40 can advance and retract with respect to the channel 103 of the endoscope 100.

Next, an operation of the distal end portion 12 will be described. Fig. 6 is a lateral cross-sectional view when the distal end portion of the medical treatment instrument 1 expands. As shown in Fig. 6, the large-diameter portion 34 can be engaged with the small-diameter portion 16. In a normal state, the inner diameter of the small-diameter portion 16 is smaller than the outer diameter of the large-diameter portion 34. However, since the needle 10 can be elastically deformed, by the width of the slit 18 being expanded in the circumferential direction of the needle 10, the inner diameter of the small-diameter portion 16 can be expanded up to the size which is approximately the same as the outer diameter of the large-diameter portion 34. For this reason, the large-diameter portion 34 can be engaged with the small-diameter portion 16.

In the embodiment, as shown in Fig. 5, the large-diameter portion 34 is positioned closer to the distal end side than the small-diameter portion 16 in an initial state. From this state, by retracting the stylet operation part 36 to pull the stylet 30 toward the proximal end side with respect to the needle 10, as shown in Fig. 6, the large-diameter portion 34 moves toward the proximal end side and is engaged with the small-diameter portion 16 so that the width of each of the slits 18 expands. When the large-diameter portion 34 is engaged with the small-diameter portion 16, an operator who operates the stylet operation part 36 feels an operation sensation (clicking sensation). Since the distal end portion 12 is divided into three portions by the slits 18 as described above, by expanding the widths of the slits 18, each of the three portions of the distal end portion 12 is elastically deformed to move toward the outside in the radial direction of the needle 10. That is, the housing portion 14 and the small-diameter portion 16 expand in the radial direction of the needle 10 so that the space 15 is open to the outside of the needle 10.

By moving the large-diameter portion 34 engaged with the small-diameter portion 16 further toward the proximal end by operating the stylet operation part 36, the engagement between the large-diameter portion 34 and the small-diameter portion 16 is released, and the large-diameter portion 34 moves toward the proximal end side of the small-diameter portion 16. As the engagement between the large-diameter portion 34 and the small-diameter portion 16 is released, each of the three portions of the distal end portion 12 is elastically deformed to move to the inside in the radial direction of the needle 10 by an elastic force attempting to restore to the original shape of the needle 10, and the width of each of the slits 18 is restored to the original size. That is, the housing portion 14 and the small-diameter portion 16 are closed, and the space 15 is closed with respect to the outside of the needle 10. In addition, in the above example, although the operation of the distal end portion 12 when the large-diameter portion 34 is moved from the distal end side toward the proximal end side of the small-diameter portion 16 has been described, the distal end portion 12 performs the same operation when the large-diameter portion 34 is moved from the proximal end side toward the distal end side of the small-diameter portion 16.

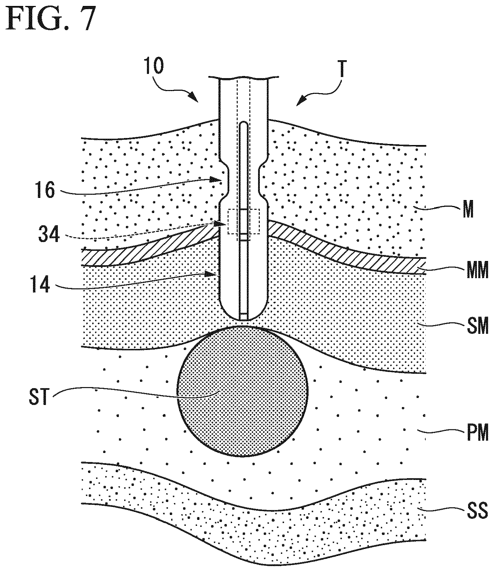

Next, treatment using the medical treatment instrument 1 according to the embodiment will be described with reference to Figs. 7 to 10. Figs. 7 to 10 are views showing an operation when the medical treatment instrument 1 is being used. Here, the above treatment of collecting tissue of a gastric submucosal tumor will be described as an example. As shown in Fig. 7, on a stomach wall, a mucosa M, muscularis mucosae MM, a submucosa SM, a proper muscle PM, and a subserosa SS are disposed in that order, and a submucosal tumor ST is formed in the proper muscle PM.

First, an operator inserts the endoscope insertion part 101 of the endoscope 100 from a patient's mouth into the body cavity, and moves the distal end of the endoscope insertion part 101 up to a portion near a target portion T. The medical treatment instrument 1 is inserted into the channel 103, and the distal end of the medical treatment instrument 1 is moved up to the distal end of the endoscope insertion part 101. Here, the distal end of the needle 10 is stored in the sheath 40. In addition, in the treatment example, a case in which the large-diameter portion 34 of the stylet 30 is arranged at the distal end side of the small-diameter portion 16 of the needle 10 in the initial state will be described.

The operator operates the sheath operation part 42 to cause the sheath 40 to protrude from the distal end of the endoscope insertion part 101 while observing the target portion T with an imaging means provided at the distal end of the endoscope insertion part 101. Then, the operator advances the needle operation part 20 to cause the needle 10 to protrude from the distal end of the sheath 40, and punctures the target portion T by the needle 10.

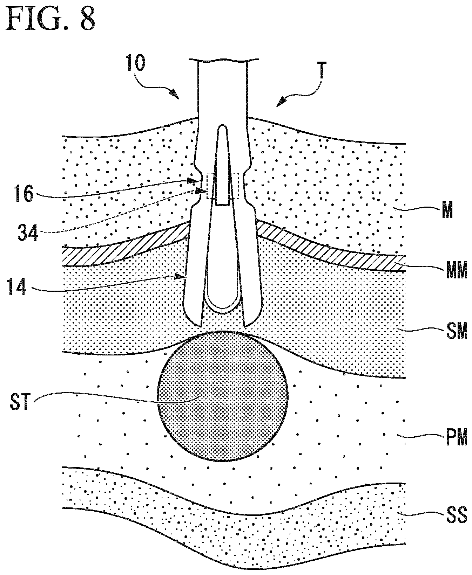

When the target portion T is punctured by the needle 10, as shown in Fig. 7, the operator first inserts the distal end of the needle 10 into the submucosa SM. Next, as shown in Fig. 8, the operator retracts the stylet operation part 36 and pulls the stylet 30 toward the proximal end side with respect to the needle 10 to move the large-diameter portion 34 toward the proximal end side. Thus, the large-diameter portion 34 is engaged with the small-diameter portion 16, and the housing portion 14 expands. Here, since the housing portion 14 is inside the mucosa M, the operator is unable to see that the housing portion 14 has expanded with the imaging means of the endoscope insertion part 101. However, the operator can determine that the housing portion 14 has expanded by feeling the clicking sensation generated due to the engagement between the large-diameter portion 34 and the small-diameter portion 16.

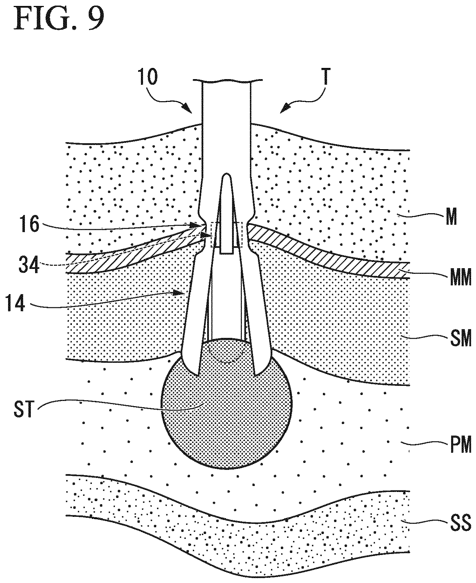

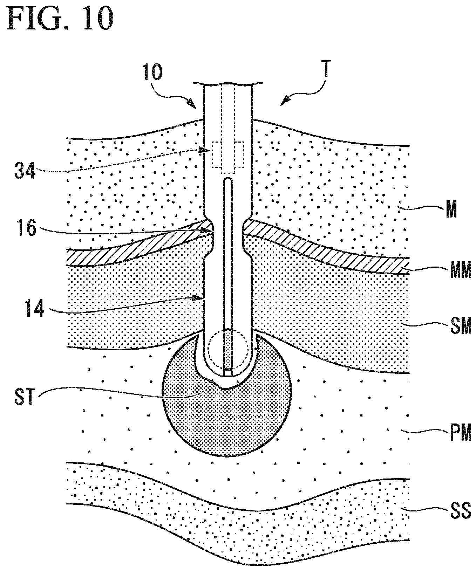

Then, in this state, the operator operates the needle operation part 20 and sticks the needle 10 into the submucosal tumor ST as shown in Fig. 9. The operator retracts the stylet operation part 36 and pulls the stylet 30 further toward the proximal end side with respect to the needle 10 to move the large-diameter portion 34 further toward the proximal end side. Thus, the engagement between the large-diameter portion 34 and the small-diameter portion 16 is released, and as shown in Fig. 10, the housing portion 14 is closed by the elastic force of the needle 10. When the housing portion 14 is closed, tissue of the submucosal tumor ST can be collected into the space 15. Here, depending on the amount of tissue being collected, there are cases in which the housing portion 14 is inflated slightly more than in the originally closed state. That is, the housing portion 14 can collect tissue of a larger volume than a volume defined by the inner diameter of the housing portion in a state in which the housing portion 14 is closed. While the housing portion 14 is closed, the operator retracts the needle operation part 20 to retract the needle 10 until the distal end of the needle 10 is stored in the sheath 40. The operator withdraws the medical treatment instrument 1 from the channel 103.

In addition, although it has been described above that the large-diameter portion 34 is arranged at the distal end side of the small-diameter portion 16 in the initial state, the large-diameter portion 34 may be arranged at the proximal end side of the small-diameter portion 16 in the initial state. In this case, the operation of moving the large-diameter portion 34 toward the proximal end side in the description above is substituted with an operation of moving the large-diameter portion 34 toward the distal end side.

In the medical treatment instrument 1 according to the embodiment, the slits 18 are provided at the distal end portion 12 of the needle 10, and the distal end portion 12 (the housing portion 14 and the small-diameter portion 16) can be elastically deformed to expand toward the outside in the radial direction. In this way, a sufficient amount of tissue can be collected even when the outer diameter of the needle 10 is narrow. In addition, in the medical treatment instrument 1 according to the embodiment, from the state in which the distal end portion 12 has expanded toward the outside in the radial direction, the distal end portion 12 is restored to the originally closed state by its elastic force. For this reason, even when collecting tissue of the submucosal tumor which is hard tissue, the distal end portion 12 can be restored to the originally closed state by its elastic force overcoming the hardness of the tissue, and the tissue can be reliably collected.

In addition, in the medical treatment instrument 1 according to the embodiment, a blade may be formed at a portion where the slit 18 of the distal end portion 12 of the needle 10 is formed. In this way, when the distal end portion 12 is closed, the tissue can be easily split and collected.

In the medical treatment instrument 1 according to the embodiment, the operator can recognize that the large-diameter portion 34 is engaged with the small-diameter portion 16 by the clicking sensation. In addition, by providing a known stopper mechanism, which stops the stylet 30 at a time when the stylet 30 has been operated by a predetermined amount, at the stylet operation part 36, the medical treatment instrument 1 may be constituted in a way that the operator can check that the large-diameter portion 34 is engaged with the small-diameter portion 16.

In the medical treatment instrument 1 according to the embodiment, the sheath 40 is not an essential constitution. The medical treatment instrument 1 may not include the sheath 40. In addition, instead of the sheath 40, a known sheath and the medical treatment instrument 1 may be used in combination.

### (Second embodiment)

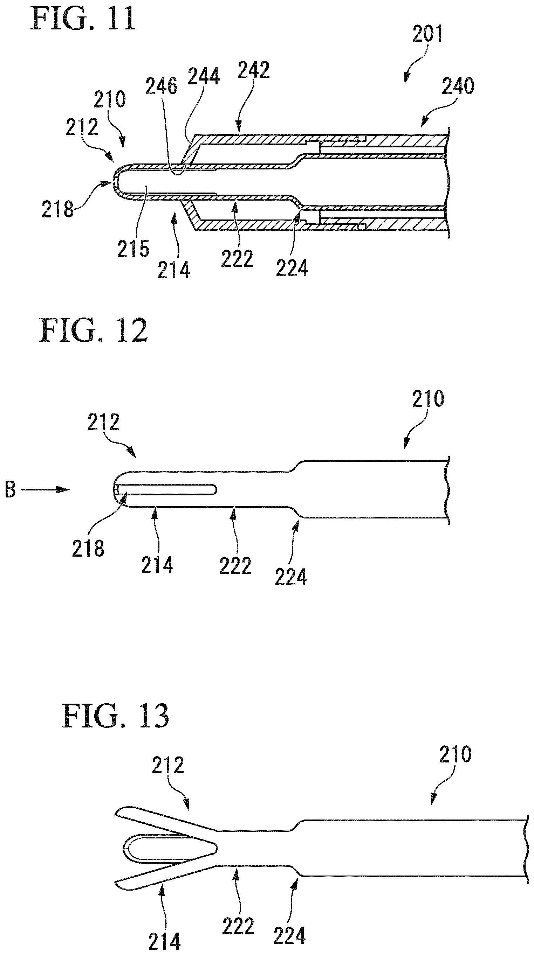

Next, a second embodiment of the present invention will be described with reference to Figs. 11 to 17. A medical treatment instrument 201 according to the embodiment is different from the medical treatment instrument 1 according to the first embodiment in that the stylet 30 is not included. Further, in the medical treatment instrument 201 according to the embodiment, constitutions of the distal end portion of the needle 10 and the distal end portion of the sheath 40 are different from those of the medical treatment instrument 1 according to the first embodiment. In addition, with respect to parts having the same constitutions as the medical treatment instrument 1 according to the first embodiment, a detailed description thereof will be omitted.

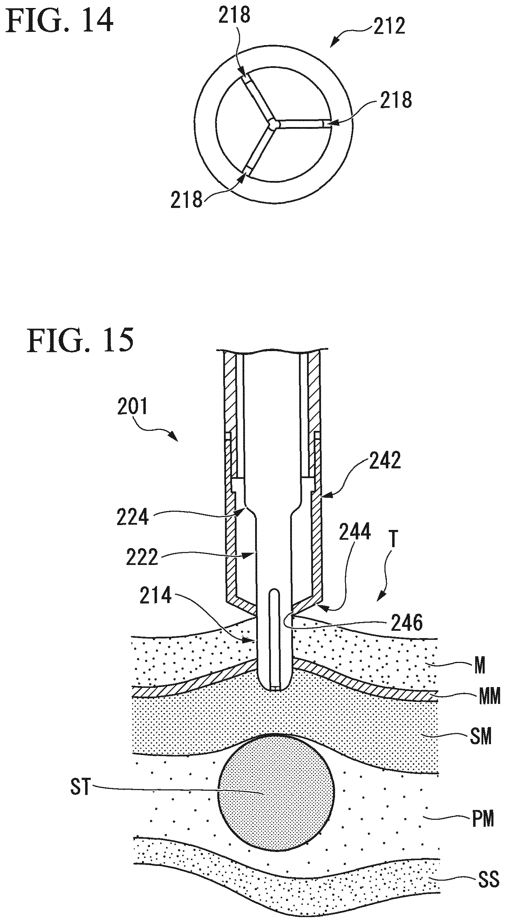

Fig. 11 is a lateral cross-sectional view of a distal end portion of the medical treatment instrument 201 according to the embodiment. Fig. 12 is a view showing a state in which a distal end portion 212 of a needle 210 of the medical treatment instrument 201 is closed. Fig. 13 is a view showing a state in which the distal end portion 212 of the needle 210 of the medical treatment instrument 201 is open. Fig. 14 is a view showing the needle 210 viewed from an arrow B in Fig. 12. As shown in Fig. 11, the medical treatment instrument 201 includes the needle 210 and a sheath 240, but does not include a stylet.

As shown in Figs. 11 and 12, the distal end portion 212 of the needle 210 includes a housing portion 214, a straight tube portion 222 provided at a proximal end of the housing portion 214, and a stepped portion 224 provided at a proximal end of the straight tube portion 222. As in the first embodiment, a space 215 capable of accommodating tissue to be collected is formed in the housing portion 214. In addition, a slit 218 which extends from the distal end to the proximal end of the housing portion 214 is formed in the housing portion 214. In the embodiment, although an example in which three slits 218 are formed is shown, the number of slits is not limited thereto as long as there are one or more slits. When the plurality of slits are formed, as in the embodiment shown in Fig. 14, the slits 218 are preferably formed at equal intervals in a circumferential direction of the needle 210. Each of the slits 218 communicates with the space 215. In addition, the slits 218 intersect with or join each other at the distal end of the needle 210. Thus, the housing portion 214 is divided into three portions by the slits 218. As shown in Fig. 13, each of the three portions of the housing portion 214 is formed in a shape that expands toward the outside in the radial direction of the needle 210 by plastic deformation. In addition, since the needle 210 can be elastically deformed like the needle 10 of the first embodiment, as shown in Fig. 11, the housing portion 214 can be elastically deformed in the closed state by a sheath distal end member 242 to be described below. As shown in Fig. 12, while the housing portion 214 is closed, the distal end of the housing portion 14 is formed in the shape of a curved surface. Thus, in this state, tissue can be easily punctured by the needle 210. An outer diameter of the housing portion 214 when the housing portion 214 is closed is properly set in consideration of the puncturing performance.

The straight tube portion 222 is disposed between the housing portion 214 and the stepped portion 224. The straight tube portion 222 has an inner diameter and an outer diameter which are respectively substantially the same as the inner diameter and the outer diameter of the housing portion 214 when the housing portion 214 is closed. The outer diameter of the straight tube portion 222 is smaller than an outer diameter of portions of the needle 210 other than the housing portion 214. The length of the straight tube portion 222 in the axial direction of the needle 210 is properly set depending on a length to which target tissue is punctured by the needle 210.

The stepped portion 224 has a shape which extends from the straight tube portion 222 while an outer diameter thereof expanding. The stepped portion 224 comes in contact with a protruding portion 244 of the sheath distal end member 242 to be described below. By the stepped portion 224 coming in contact with the protruding portion 244, the needle 210 is prevented from moving further toward the distal end side with respect to the sheath 240.

The sheath distal end member 242 is mounted at a distal end of the sheath 240. The sheath distal end member 242 is formed in a tubular shape having an inner diameter and an outer diameter which are respectively substantially the same as the inner diameter and the outer diameter of the sheath 240. A lumen of the sheath distal end member 242 communicates with a lumen of the sheath 240, and the needle 210 is inserted into the lumen of the sheath distal end member 242. The needle 210 can advance and retract with respect to the sheath 240 and the sheath distal end member 242. The protruding portion 244 which protrudes toward the inside in the radial direction of the sheath distal end member 242 is provided at the distal end of the sheath distal end member 242. At the distal end of the sheath distal end member 242, an opening 246 which communicates with the lumen of the sheath distal end member 242 is formed further toward the inside in the radial direction than the protruding portion 244. The diameter of the opening 246 is slightly larger than the outer diameter of the straight tube portion 222, and is smaller than the outer diameter of the stepped portion 224. Thus, although the straight tube portion 222 can be inserted into the opening 246, the stepped portion 224 cannot be inserted into the opening 246 due to coming in contact with the protruding portion 244. By being elastically deformed in the closed state, the housing portion 214 can be inserted into the opening 246. That is, by the housing portion 214 being inserted into the opening 246, an outer surface of the housing portion 214 is pressed by the protruding portion 244, and the housing portion 214 is elastically deformed in the closed state.

Next, treatment using the medical treatment instrument 201 according to the embodiment will be described using Figs. 15 to 17. Figs. 15 to 17 are views for describing an operation when the medical treatment instrument 201 is being used. As in the first embodiment, the treatment of collecting tissue of a gastric submucosal tumor will be described as an example.

First, as in the first embodiment, the operator uses the endoscope 100 to send the distal end of the medical treatment instrument 201 to a portion near the target portion T. The operator advances the needle operation part 20 to cause the needle 210 to protrude from the distal end of the sheath distal end member 242. Here, as shown in Fig. 15, a part of the housing portion 214 is arranged in the opening 246, and the housing portion 214 is closed by the protruding portion 244. In this state, the operator punctures the target portion T with the needle 210.

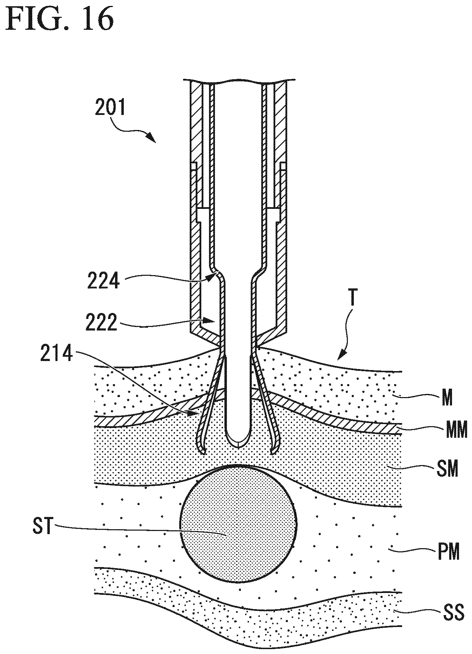

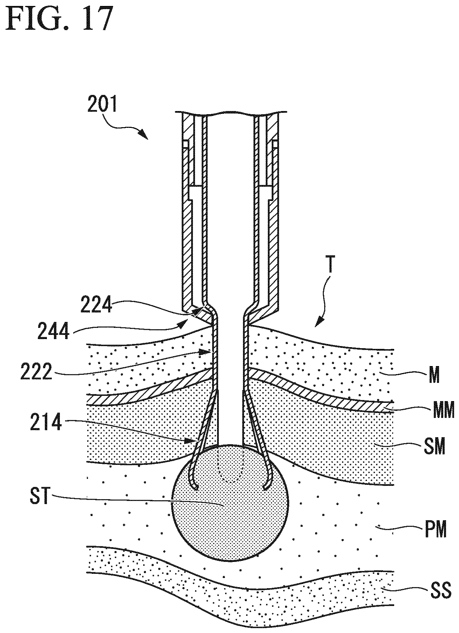

Next, the operator advances the needle 210 with respect to the sheath distal end member 242. In this way, when the housing portion 214 passes the protruding portion 244 and moves toward the distal end side, as shown in Fig. 16, the housing portion 214 is restored to the originally expanded state while pushing circumferential tissue by an elastic force attempting to restore to the original shape of the housing portion 214. In this state, as shown in Fig. 17, the operator further advances the needle 210 with respect to the sheath distal end member 242 until the stepped portion 224 comes in contact with the protruding portion 244. In this way, tissue of the submucosal tumor ST is accommodated in the space 215 of the housing portion 214. When the operator retracts the needle 210 with respect to the sheath distal end member 242, the outer surface of the housing portion 214 is pressed by the protruding portion 244, and thus the housing portion 214 is closed by elastic deformation. In this way, the tissue accommodated in the space 215 can be collected. Then, the needle 210 is withdrawn from the target portion T, and the medical treatment instrument 201 is withdrawn from the body.

In the medical treatment instrument 201 according to the embodiment, the slits 218 are provided at the housing portion 214 of the needle 210, the housing portion 214 is formed by plastic deformation in the shape that expands toward the outside in the radial direction, and the housing portion 214 can be closed by elastic deformation. Accordingly, after tissue is punctured by the needle 210 while the housing portion 214 is closed, the housing portion 214 is expanded, and thus a sufficient amount of tissue can be collected even when the outer diameter of the needle 210 is narrow. In addition, in the medical treatment instrument 1 according to the embodiment, the housing portion 214 is closed due to the outer surface of the housing portion 214 being pressed by the protruding portion 244 of the sheath distal end member 242. Accordingly, even when collecting tissue of a submucosal tumor which is hard tissue, the housing portion 214 can be closed with a force overcoming the hardness of the tissue, and the tissue can be reliably collected.

Although preferred embodiments of the present invention have been described above, the present invention is not limited to the embodiments. Additions, omissions, substitutions, and other modifications can be made without departing from the scope of the present invention. The present invention is not limited by the description above, and is only limited by the scope of the appended claims.

### Industrial Applicability

According to the medical treatment instrument according to each of the embodiments, since the housing portion of the needle can expand in the radial direction of the needle by elastic deformation, even when the outer diameter of the needle is narrow to an extent that the needle can properly puncture tissue, a sufficient amount of the tissue can be collected by the expansion of the housing portion.

### Reference Signs List

1, 201: medical treatment instrument
10, 210: needle
12, 212: distal end portion
14, 214: housing portion
16: small-diameter portion
18, 218: slit
30: stylet
34: large-diameter portion
40, 240: sheath

## Claims

1. A medical treatment instrument comprising:
a sheath;
a needle formed in a tubular shape and inserted into the sheath such that the needle is capable of advancing and retracting; and
a stylet having a first outer diameter and inserted into the needle such that the stylet is capable of advancing and retracting, wherein
a distal end portion of the needle is elastically deformable,
the distal end portion of the needle includes:
a housing portion having a space formed therein;
a small-diameter portion provided at a proximal end of the housing portion and having an inner diameter smaller than an inner diameter of the housing portion; and
a slit extending at least from a distal end of the housing portion to a proximal end of the small-diameter portion and communicating with the space,
a distal end portion of the stylet includes a large-diameter portion having a second outer diameter which is larger than the first outer diameter, and
by the large-diameter portion being engaged with the small-diameter portion, the small-diameter portion and the housing portion are elastically deformed to expand in a radial direction of the needle while a width of the slit expands.

2. The medical treatment instrument according to claim 1, wherein
the first outer diameter is smaller than the inner diameter of the small-diameter portion, and
the second outer diameter is larger than the inner diameter of the small-diameter portion and is smaller than the inner diameter of the housing portion.

3. The medical treatment instrument according to claim 1, wherein the slit comprises a plurality of slits.

4. The medical treatment instrument according to claim 1, wherein the large-diameter portion is positioned closer to a distal end side than the small-diameter portion in an initial state, and the large-diameter portion expands the small-diameter portion by the stylet being pulled toward a proximal end side with respect to the needle.
